Europäisches Patentamt

European Patent Office

Office européen des brevets

(11) Veröffentlichungsnummer: **0 268 974 B1**

(19)

(12) **EUROPÄISCHE PATENTSCHRIFT**

(45) Veröffentlichungstag der Patentschrift: **17.06.92**

(51) Int. Cl.5: **C07H 13/06**, B01F 17/00

(21) Anmeldenummer: **87116918.1**

(22) Anmeldetag: **17.11.87**

(54) **Verfahren zur Herstellung von Gemischen aus mono- und oligomeren Kohlenhydratestern, die so erhältlichen Kohlenhydratestergemische und ihre Verwendung.**

(30) Priorität: **21.11.86 DE 3639878**

(43) Veröffentlichungstag der Anmeldung:
**01.06.88 Patentblatt 88/22**

(45) Bekanntmachung des Hinweises auf die
Patenterteilung:
**17.06.92 Patentblatt 92/25**

(84) Benannte Vertragsstaaten:
**AT BE CH DE FR GB IT LI NL SE**

(56) Entgegenhaltungen:
**FR-A- 2 205 504**
**US-A- 3 551 464**

(73) Patentinhaber: **HOECHST AKTIENGESELL-
SCHAFT**
**Postfach 80 03 20**
**W-6230 Frankfurt am Main 80(DE)**

(72) Erfinder: **Deger, Hans-Matthias, Dr.**
**Am Rheingauer Weg 8**
**W-6238 Hofheim am Taunus(DE)**
Erfinder: **Fritsche-Lang, Wolfram, Dr.**
**Rhönstrasse 7**
**W-6140 Bensheim(DE)**
Erfinder: **Reng, Alwin**
**Im Schulzehnten 22**
**W-6233 Kelkheim (Taunus)(DE)**
Erfinder: **Schlingmann, Merten, Dr.**
**Schneidhainer Strasse 32a**
**W-6240 Königstein (Taunus)(DE)**
Erfinder: **Lawson, Christopher John**
**4 Plumtrees Earley**
**Reading RG6 2OO, Berkshire(GB)**

## Beschreibung

Die Erfindung betrifft ein Verfahren zur Herstellung von neuen Gemischen aus mono- und oligomeren Kohlenhydratestern durch Umsetzung von Kohlenhydraten mit gesättigten Fettsäuren und/oder esterbildenden Derivaten von Fettsäuren in flüssigem Fluorwasserstoff, der gleichzeitig als Lösungsmittel verwendet wird und am Ende der Reaktion abdestilliert wird. Die Erfindung betrifft gleichermaßen die so hergestellten neuen Kohlenhydratestergemische sowie deren Verwendung als Tenside oder Fettersatzstoffe.

Es ist bekannt, daß die Veresterung von Kohlenhydraten unter dem katalytischen Einfluß von Säuren oder Basen stattfinden kann. Diese Umsetzung ist jedoch mit verschiedenen Problemen verbunden.

In Gegenwart von Basen, wie zum Beispiel Natriummethanolat, lassen sich nur solche Kohlenhydrate verestern, die keine reduzierenden Eigenschaften besitzen (z.B. Saccharose oder Trehalose) oder Kohlenhydrat-Derivate, deren reduzierende Endgruppen geschützt bzw. beseitigt worden sind, z.B. O-Glycoside wie $\alpha$-Methylglucosid oder Lactitol etc. In diesen Fällen werden für die Acylierung in der Regel Umesterungsbedingungen angewendet, d.h. aus der Mischung von Kohlenhydrat und Fettsäureester wird unter Anwendung hoher Temperaturen und Vakuum die leicht flüchtige Alkoholkomponente (meist Methanol oder Ethanol) des Acylierungsmittels ausgetrieben (vgl. Ullmann's Enzyklopädie der techn. Chemie, 4. Auflage, Bd. 22, Seite 495 ff., 1982). Unter derart drastischen Bedingungen (100-150°C) werden aber reduzierende Kohlenhydrate in beträchtlichen Ausmaß karamelisiert bzw. zersetzt. Selbst nichtreduzierende Kohlenhydrate erleiden deutliche Dunkelfärbungen, so daß die zersetzten Produkte später mit großem Aufwand bei der Aufarbeitung wieder entfernt werden müssen. Die Reaktion selbst kann bekanntermaßen sowohl lösungsmittelfrei als auch in inerten Lösungsmitteln durchgeführt werden. Die lösungsmittelfreie Reaktion, d.h. die Reaktion in der Schmelze, erfordert erhöhte Temperaturen, die in nicht zu vernachlässigendem Umfang zu Karamelisierungen und Zersetzungen führen. Bei Verwendung von inerten Lösungsmitteln können aufgrund der unterschiedlichen Polarität der Reaktanten im allgemeinen nur dipolar-aprotische Lösungsmittel eingesetzt werden, wie Dimethylformamid (DMF), Dimethylsulfoxid (DMSO), Hexamethylphosphorsäuretriamid (HMPT), Pyridin etc., die toxikologisch bedenklich sind und vor allem Rückstandsprobleme mit sich bringen.

In der amerikanischen Patentschrift US-PS 3,551,464 ist ein Veresterungsverfahren für mehrwertige Alkohole mit Fettsäuren beschrieben, das die Verwendung von flüssigem Fluorwasserstoff sowohl als Lösungsmittel und Katalysator als auch als wasserbindendes Medium beinhaltet. In dem genannten Patent werden verschiedene mehrwertige Alkohole (Glycerin, Trimethylolpropanol etc.) mit gesättigten Fettsäuren oder Ölsäure bzw. deren Estern zu überwiegend Monoester enthaltenden Produkten umgesetzt.

Es ist ferner bekannt, daß unter der Einwirkung von Säuren glycosidische Bindungen nicht stabil sind, so daß beim Einsatz von Di- bzw. Oligosacchariden stets mit der Spaltung der glycosidischen Bindungen und daraus resultierenden Folgeprodukten zu rechnen ist.

Es ist weiterhin bekannt, daß Fluorwasserstoff ein ausgezeichnetes Lösungsmittel sowohl für Kohlenhydrate aller Art (z.B. auch Cellulose) als auch für langkettige Fettsäuren ist. In diesem Medium werden Kohlenhydrate wie Glucose in entsprechende C-1-Fluoride überführt, die je nach den angewendeten Bedingungen als solche isoliert oder in Oligosaccharide (Rückkondensation oder Reversion) übergeführt werden können (vgl. Defaye, Gadelle und Pedersen, Carbohydr. Res. Bd. 110 (1982), 217-227). Die Carbonsäuren werden in Gegenwart von Fluorwasserstoff in die reaktiven Säurefluoride umgewandelt.

Sowohl die Veresterung von Polyalkoholen (US-PS 3,551,464) als auch die Rückkondensation von Kohlenhydraten ist in flüssigem Fluorwasserstoff von Gleichgewichten gekennzeichnet, deren Lage von den verschiedensten Einflußgrößen, wie Konzentration, Temperatur, Molverhältnisse der Reaktanten, abhängen.

Erfindungsgemäß werden nun Kohlenhydrate mit gesättigten Fettsäuren und/oder deren Derivaten in flüssigem Fluorwasserstoff umgesetzt.

Wie aus der Literatur entnommen werden kann (loc. cit. Defaye et al.) ist auf Grund der loc. cit. kinetischen Daten belegt, daß die Fluoridierung von Glucose bzw. die Hydrofluorolyse von Stärke eine sehr schnelle Reaktion ist, so daß zu erwarten war, daß von den beiden Gleichgewichtsreaktionen der Veresterung und der Reversion, (Rückkondensation), die letztere stärker begünstigt wird.

Es wurde nun überraschend gefunden, daß im Reaktionsmedium des erfindungsgemäßen Verfahrens im wesentlichen dem Gleichgewicht der Veresterung der Vorrang vor der Rückkondensation gegeben wird, so daß auf diese Weise Gemische von mono- und oligomeren Kohlenhydratestern hergestellt werden können.

Die Erfindung betrifft somit ein Verfahren zur Herstellung von Gemischen aus mono- und oligomeren Kohlenhydratestern durch Umsetzung von reduzierenden oder nicht reduzierenden Kohlenhydraten mit einer oder mehreren, sich in der Anzahl der C-Atome in der linearen oder verzweigten Alkylkette unterscheidenden, gesättigten Fettsäuren und/oder Fettsäureanhydriden und/oder Fettsäurehalogeniden und/oder Fettsäureestern , das dadurch gekennzeichnet ist, daß man die Umsetzung in flüssigem Fluorwasserstoff als Reaktionsmedium und Lösungsmittel durchführt, und anschließend den Fluorwasserstoff abdestilliert.

Bei dieser Reaktion entstehen Produktgemische, die sowohl mono- als auch oligomere Kohlenhydratester enthalten, wobei die Ester auch aus Fettsäureestern mit unterschiedlich langen Alkylketten bestehen können.

Die Erfindung betrifft ebenfalls die neuen Produktgemische, die durch das erfindungsgemäße Verfahren erhältlich sind, die entsprechend ihrer Zusammensetzung sowohl als Tenside als auch z.B. im Lebensmittelbereich als Fettersatzstoff eingesetzt werden können.

Bei dem erfindungsgemäßen Verfahren werden die Reaktanten in flüssigem Fluorwasserstoff, der auch als wasserentziehendes Mittel wirkt, gelöst und bei Temperaturen von mindestens -40°C, bevorzugt mindestens -20°C, insbesondere mindestens 0°C, höchstens jedoch +80°C, bevorzugt höchstens +60°C, insbesondere höchstens +40°C umgesetzt. Die Reaktionszeit beträgt mindestens 10 Minuten, bevorzugt mindestens 30 Minuten, insbesondere mindestens eine Stunde; eine Reaktionszeit von 6 Stunden, bevorzugt 3 Stunden, insbesondere 2 Stunden, sollte jedoch nicht überschritten werden, wobei die Zeit für das Abdestillieren des Fluorwasserstoffs nicht mit eingerechnet ist.

Durch das erfindungsgemäße Vorgehen wird erreicht, daß die Veresterung in homogener Phase und unter milden Bedingungen ablaufen kann.

Als Kohlenhydrate sind erfindungsgemäß Aldohexosen, bevorzugt Galactose oder Mannose, insbesondere Glucose und davon abgeleitete Oligo- und Polysaccharide sowie insbesondere deren feste, trockene Pulvermischungen einsetzbar. Auch Cellulose oder Stärke sind als Ausgangsmaterial geeignet. Weiterhin sind für das erfindungsgemäße Verfahren auch oligo- und polymere Kohlenhydrate geeignet, die Aldohexosen als Bausteine enthalten, wie z.B. die Saccharose. Ketohexosen, wie D-Fructose, reagieren in Fluorwasserstoff nicht zu Oligofructosanen, sondern zu einem nichtreduzierenden Dianhydro-difructosid-Anomerengemisch aus pyranoiden bzw. furanoiden Teilstrukturen (vgl. Defaye, Gadelle und Pedersen, Carbohydr. Res. Bd. 136 (1985), 53-65), welche sich aber ihrerseits mit Hilfe des erfindungsgemäßen Verfahrens ebenfalls durch Veresterung in flüssigem Fluorwasserstoff zu neuen Produkten umsetzen lassen.

Als Fettsäuren zur Herstellung der erfindungsgemäßen Produkte kommen gesättigte lineare oder verzweigte Monocarbonsäuren zur Anwendung. Es ist auch möglich, zwei oder mehrere unterschiedliche Monocarbonsäuren, die voneinander verschieden lange Alkylreste (Kohlenstoffketten) aufweisen oder sich bei gleicher C-Atomzahl in der Art ihrer Verzweigung unterscheiden, einzusetzen.

Erfindungsgemäß können also die Kohlenhydrate mit

a) nur einer Monocarbonsäure oder

b) mindestens zwei sich in der Art ihrer Verzweigung unterscheidenden, jedoch die gleiche C-Atomzahl aufweisenden Monocarbonsäuren oder

c) mindestens zwei sich in der Anzahl der C-Atome in der Alkylkette unterscheidenden oder

d) mindestens zwei sich sowohl in der Anzahl der C-Atome in der Alkylkette als auch in der Art ihrer Verzweigung unterscheidenden Monocarbonsäuren

umgesetzt werden. Die Alkylketten der Monocarbonsäuren können auch mehrfach verzweigt sein.

Es können ferner auch Fettsäure-Ester, vorzugsweise solche von Glycerin oder niederen, leicht flüchtigen Alkoholen, das sind solche mit bis zu 3 C-Atomen, also Methanol, Ethanol, n-Propanol oder i-Propanol sowie die Säurehalogenide, insbesondere die Säurechloride oder die Säureanhydride eingesetzt werden. Bevorzugt kommen Fettsäuren und/oder Fettsäureester und/oder Fettsäurehalogenide und/oder Fettsäureanhydride mit einer Kettenlänge von 6 bis 22 C-Atomen, insbesondere solche mit $C_{12}$-$C_{18}$-Kette zur Anwendung. Besonders bevorzugt aufgrund ihrer Bioverträglichkeit sind solche mit gerader Kohlenstoffatomanzahl. Im Falle der Ester kann die Reaktion als Umesterung beschrieben werden.

Das Molverhältnis der eingesetzten Reaktanten (Kohlenhydrate:Fettsäure) bestimmt nicht nur, wie eigentlich erwartet, den Veresterungsgrad der Produkte, sondern regelt auch die Kohlenhydrat-Zusammensetzung, d.h. den Oligomerisierungsgrad (DP) des Kohlenhydrat-Grundgerüsts. Eine Untersuchung der erhaltenen Produkte zeigte, daß z.B. aus einem Einsatzverhältnis Glucose/Stearinsäure von 1:5 nicht ein hochacyliertes Oligosaccharid entsteht, sondern eine Mischung aus peracyliertem Mono-, Di- und Trisaccharid. Bei einem Verhältnis der Ausgangsstoffe von 1:1 wird ein partiell acyliertes Oligosaccharid mit einem mittlerem Oligomerisierungsgrad (DP) von 3 - 10 gefunden, während bei einem Verhältnis von 10:1 ein Polysaccharidgemisch mit nur vereinzelten Acylgruppen gebildet wird. Erfindungsgemäß kann das Molverhältnis der eingesetzten Reaktanten (Kohlenhydrat:Fettsäure) von 10:1 bis 1:5 variiert werden. Auch Abweichungen von diesen Grenzverhältnissen nach oben und nach unten sind möglich. Eine Erhöhung des Fettsäureanteils auf über 1:5 führt zu keinen wesentlichen Veränderungen mehr, da die stöchiometrische Grenze für die mögliche Veresterung der vorhandenen Hydroxylgruppen erreicht ist.

Das erfindungsgemäße Verfahren wird vorteilhaft wie folgt durchgeführt:

1. Herstellung einer Lösung der entsprechend gewählten Ausgangsstoffe (Art der Kohlenhydrate und der Fettsäure bzw. Fettsäurederivate sowie Molverhältnis der Reaktanten) in flüssigem Fluorwasserstoff, wobei die Gesamtkonzentration der Ausgangsstoffe 10-60 Gew.-%, vorzugsweise 25-35 Gew.-%, bezogen auf die Fluorwasserstofflösung, beträgt.

2. Einhalten einer Reaktionszeit von mindestens 10 Minuten und höchstens 6 Stunden. Dabei soll die Temperatur mindestens -40°C, höchstens jedoch 80°C, betragen. Bei den angegebenen Reaktionszeiten und -temperaturen handelt es sich um Richtwerte, die ggf. auch über- oder unterschritten werden können.

3. Abdestillieren des Fluorwasserstoffs, gegebenenfalls unter vermindertem Druck, mit oder ohne Schutz- oder Trägergas. Dabei kann der Fluorwasserstoff in einer gekühlten Vorlage zur Wiederverwendung aufgefangen werden.

Zur Aufarbeitung der Reaktionsprodukte können sich je nach Produktzusammensetzung einzelne oder mehrere der folgenden weiteren Reaktionsschritte anschließen:

4. Behandeln des hinterbleibenden festen, meist leicht schmelzenden Rückstandes mit geeigneten Lösungsmitteln, in der Regel $H_2O$, in besonderen Fällen DMF oder Dioxan. Anschließend wird zur Neutralisation und Entfernung der im Rückstand enthaltenen Menge restlichen Fluorwasserstoffs z.B. mit geeigneten Ionenaustauschern oder mit Calciumhydroxid oder Calciumcarbonat versetzt. Die Lösung wird dann vom gebildeten Calciumfluorid abgetrennt, z. B. abfiltriert oder zentrifugiert.

5. Gewinnung des Produktes aus der Lösung durch Einengung der Lösung unter vermindertem Druck mit Hilfe eines Rotationsverdampfers oder Dünnschichtverdampfers, ggf. durch Fällung des Reaktionsproduktes mittels geeigneter Fällungsmittel und anschließender Filtration des Niederschlags. Als Fällungsmittel kommen bevorzugt Aceton, Ether oder Hexan in Betracht.

6. Gegebenenfalls eine chromatographische Trennung zur Entfernung nicht umgesetzter Fettsäure.

Durch die chromatographische Trennung gelingt es -insbesondere für Anwendungen im Lebensmittelbereich - den freien Fettsäuregehalt auf unter 0,1-0,3 % zu senken.

Bei dem erfindungsgemäßen Verfahren wird zunächst eine helle bis gelblich gefärbte Lösung des Reaktionsproduktes in flüssigem Fluorwasserstoff erhalten. Zur Isolierung des Reaktionsproduktes wird der Fluorwasserstoff, am einfachsten durch Destillation, zweckmäßig so weit wie möglich, entfernt. Nach dem Abdestillieren des Fluorwasserstoffs hinterbleibt ein meist leicht schmelzender, mit zunehmender Fettsäurekettenlänge intensiver gefärbter, fester Rückstand, der je nach Veresterungsgrad, entweder in wäßriger oder Acetonlösung mit Calciumcarbonat neutralisiert werden kann. In Ausnahmefällen, nämlich zur Gewinnung wasserlöslicher Kohlenhydratester, ist die Verwendung von DMF oder anderen dipolar-aprotischen Lösungsmitteln erforderlich bei der Aufarbeitung.

Das nach dem beschriebenen Verfahren hergestellte Produkt ist als farbloses oder schwach gelbliches Pulver oder Wachs oder als Sirup erhältlich. Durch die Ausfällung als unlösliches Calciumfluorid und/oder die Anwendung wirksamer Ionenaustauscher gelingt es, den Restfluorgehalt (hauptsächlich als $CaF_2$ vorliegend) im Produkt auf einen Wert unter 20 ppm zu reduzieren.

Die erfindungsgemäß hergestellten Produkte lassen sich durch Anwendung verschiedener Methoden bezüglich ihrer Struktur und Zusammensetzung untersuchen. Zur Charakterisierung haben sich die für die Fettanalytik bekannten Kennzahlenbestimmungen als geeignet erwiesen (vgl. Handbuch der Lebensmittelchemie, Bd. 4, S. 551 ff., Springer-Verlag 1969). Insbesondere sind die Bestimmungen der Säurezahl, Hydroxylzahl und der Verseifungszahl bei den erfindungsgemäßen Produkten angewendet worden. Für die Bestimmung der Verseifungszahl bzw. des Äquivalentgewichtes von Estern ist allerdings eine Reduktion der reduzierenden Endgruppen erforderlich.

Als weitere Analysenmethode kann die Gaschromatographie herangezogen werden. Hiermit ist es möglich, insbesondere bei niedrigen Oligomerisierungsgraden bzw. niedrigen Veresterungsgraden, Information über die Zusammensetzung zu erhalten.

Eine Aussage über das Ausmaß der Rückreaktion gewinnt man dadurch, daß nach katalytischer Hydrierung der Probe und alkalischer Hydrolyse der Estergruppen die freigesetzten Kohlenhydrate mittels geeigneter Methoden, z.B. Hochdruckflüssigkeitschromatographie (HPLC) analysiert werden. Die Untersuchung von Produkten, die durch Umsetzung von Glucose mit Stearinsäure erhalten wurden, haben ergeben, daß durch Erhöhung des Fettsäureanteils bei den Reaktanten ein geringerer Grad der Oligomerisierung im Produkt verursacht wird.

NMR-spektroskopische Untersuchungen ($^1$H und $^{13}$C) ergeben aufgrund der heterogenen Zusammensetzung der Produkte in Bezug auf mono- oder oligomere Kohlenhydrateinheiten und deren Verknüpfungsarten sowie der jeweiligen Bildungsmöglichkeiten von Mono- oder höheren Estern keine zusätzlichen Interpretationsmöglichkeiten.

4

EP 0 268 974 B1

Die vorliegende Erfindung betrifft außerdem die Verwendung der erfindungsgemäßen Gemische aus mono- und oligomeren Kohlenhydratestern als oberflächenaktive Verbindungen. Der entsprechende Anwendungsbereich hängt wesentlich von der Natur des eingesetzten Kohlenhydrats ab, sowie insbesondere vom Grad der Veresterung. So können die erfindungsgemäßen Gemische aus mono- und oligomeren Kohlenhydratestern wasserlöslich (hydrophil) oder fettlöslich (lipophil) sein und daher sowohl schaumaktive oder verdickende Eigenschaften als auch emulgierende Eigenschaften besitzen. Als grobes Maß für die Lipophilie bzw. Hydrophilie der erfindungsgemäßen Produktgemische kann die Hydroxylzahl herangezogen werden, wobei Werte über ca. 200-250 (abhängig von der verwendeten Fettsäure und dem Oligomerisierungsgrad) Produkten mit hydrophilem Charakter entsprechen, während bei Werten unter 200-250 lipophile Produkte vorliegen. In Abhängigkeit von diesen Produkteigenschaften kommen als Einsatzgebiet kosmetische und technische Bereiche in Betracht. Bei entsprechender Zusammensetzung und der damit verbundenen physiologischen Unbedenklichkeit bieten sich auch Einsatzmöglichkeiten für lebensmitteltechnische Zwekke, z.B. als Fettersatzstoffe an. Besonders geeignet sind die peracylierten Gemische aus mono- und oligomeren Kohlenhydraten oder solche mit einem hohen Veresterungsgrad. Bei der letzteren Anwendung ergibt sich am Beispiel ausgewählter Lebensmittel, bei vollständigem Ersatz des darin enthaltenen Fettes durch die erfindungsgemäßen Produkte, eine Einsparung an Kalorien schon allein dadurch. daß der im Produkt enthaltene Kohlenhydratanteil einen niedrigeren kalorischen Brennwert besitzt als der Fettanteil (s. Beispiel 14 Tabelle 1/2).

Gleichermaßen günstig ist die bei den erfindungsgemäßen Produktgemischen vorhandene hohe biologische Abbaubarkeit, wie sie auch für die im Lebensmittelbereich zugelassenen Ester, z.B. Sorbitanester oder Saccharoseester, bekannt ist. Diese Eigenschaft der erfindungsgemäßen Produktgemische resultiert letztlich aus ihrer Naturverwandschaft. So sind z.B. die Produktgemische im Falle der Verwendung von Fettsäuren mit gerader Kohlenstoffatomanzahl ausschließlich aus in der Natur vorkommenden Komponenten aufgebaut. Dies ist vor allem für den Einsatz in kosmetischen Präparaten von Bedeutung, da dort in zunehmendem Maße der sogenannte Naturcharakter herausgestellt wird.

Die erfindungsgemäßen Produktgemische eignen sich aufgrund ihrer emulgierenden bzw. koemulgierenden Wirkung besonders zur Herstellung von kosmetischen und pharmazeutischen Emulsionen mit speziellen anwendungstechnischen Eigenschaften. In an sich bekannter Weise lassen sich mit den erfindungsgemäßen Produktgemischen flüssige und cremeförmige O/W-Emulsionen (Öl/Wasser-Emulsion) formulieren. Durch den Zusatz der erfindungsgemäßen Produktgemische wird ferner auch die physikalische Lagerstabilität von O/W-Emulsionen verbessert, d.h. die beschriebene Formulierung weist eine Stabilität von über 2 Monaten bei +45°C Lagertemperatur auf. Im Gegensatz zu den üblicherweise eingesetzten Emulgatoren und Konsistenzgebern können die erfindungsgemäßen Produktgemische auch als Substitute für die in kosmetischen Präparate eingesetzten Öl- und Fettkomponenten, z.B. Paraffinöle, Triglyceride und u.ä. verwendet werden. Ein besonderer Vorteil der erfindungsgemäßen Produktgemische ist ihre Oxidationsstabilität im Vergleich zu herkömmlichen Triglyceriden nativer Basis. Für die Praxis bedeutet dies, daß die entsprechenden Fertigformulierungen in Gegenwart von Luftsauerstoff stabil sind und nach längerer Lagerzeit bzw. Anwendung keinen unangenehmen ranzigen Geruch aufweisen.

Ein weiterer Vorteil bei der Verwendung dieser erfindungsgemäßen Produktgemische ist ihre bifunktionelle Eigenschaft, d.h. ihre fettende und emulgierende Wirkung zugleich. Dies führt zu einer Einsparung bzw. Verringerung der üblicherweise benötigten Emulgatoren, da die erfindungsgemäßen Produktgemische bereits eine eigene Emulgierwirkung aufweisen. Aufgrund dieser bifunktionellen Eigenschaft (two-in-one-Verbindungen) kann bei Verwendung der erfindungsgemäßen Produktgemische in kosmetischen oder pharmazeutischen Präparaten, die auf die Haut aufgetragen werden, die Belastung der Haut mit zusätzlichen grenzflächenaktiven Substanzen verringert werden. Die Herstellung einer Testemulsion durch Einarbeitung der erfindungsgemäßen Produktgemische ist in Beispiel 14 beschrieben, das jedoch lediglich erläuternden Charakter hat. Ein wesentlicher Parameter für die Stabilisierung, d.h. die Konsistenz von Emulsionen ist dabei die Erhöhung der Viskosität in den Anwendungsemulsionen, wie er im gegebenen Beispiel beobachtet werden kann.

Im Beispiel 15, das ebenfalls lediglich zur näheren Erläuterung dient, wird die Verwendung der erfindungsgemäßen Produktgemische bei der Herstellung einer O/W-Emulsion für kosmetische oder pharmazeutische Hautpflege beschrieben.

Die vorliegende Erfindung bezieht sich somit auch auf neue Konsistenzgeber mit emulgierender bzw. koemulgierender Wirkung zur Herstellung von O/W-Emulsionen. Da die Produktgemische durch Umsetzung von natürlichen Kohlenhydraten mit natürlichen Fettsäuren synthetisiert werden können, eignen sich die so erhältlichen Produktgemische insbesondere zur Herstellung von kosmetischen und pharmazeutischen O/W-Emulsionen, wobei diese die erfindungsgemäßen Produktgemische in einer Menge von 0,1 bis 15 Gew.-%, bevorzugt in einer Menge von 0,5 bis 8 Gew.-%, bezogen auf die gesamte Zubereitung enthalten.

5

Die Erfindung wird durch die nachstehenden Beispiele näher erläutert. Darin bedeutet der Begriff "Vakuum" stets das der Wasserstrahlpumpe.

Beispiel 1

Die Reaktion wird in einer Polytetrafluorethylen-(PTFE)-Destillationsapparatur durchgeführt, bestehend aus 650 ml Rundkolben, 50 cm-Kolonne (ohne Füllkörper), Destillationsaufsatz, Liebig-Kühler (Luftkühlung genügt), Vakuumvorstoß und 650 ml-Vorlage (auf -60°C gekühlt).

Zu dem vorgelegten, auf 0°C vorgekühlten, flüssigen Fluorwasserstoff (355 g) werden unter Rühren 76,1 g wasserfreie Glucose und 76,1 g Stearinsäure (Molverhältnis 1,58:1) gegeben und das Gemisch 1 Stunde bei 20°C gerührt (Magnetrührung). Unter Erwärmen auf 50-60°C und Rühren wird bei langsam sinkendem Druck der Fluorwasserstoff abdestilliert, der Hauptteil bei vollem Wasserstrahlvakuum. Nach 1,5-2,5 Stunden sind etwa 90-95% des eingesetzten Fluorwasserstoffs entfernt. Man erhält einen wachsartigen, rosagefärbten Rückstand. Dieser wird in 400 ml 70°C warmem Wasser aufgenommen, durch Gießen auf Eis wieder verfestigt und bei Raumtemperatur abgesaugt. Der Feststoff wird in 1,5 1 Aceton gelöst und mit 100 g festem Calciumcarbonat bis zur neutralen Reaktion rückfließend erhitzt (1-2 Stunden). Anschließend wird das ausgefällte Calciumfluorid abfiltriert und die Lösung im Vakuum eingeengt. Man erhält 95,2 g eines farblosen, wachsartigen Feststoffes.

Schmelzbereich: 58,0-60,5°C
Oberflächenspannung: $\sigma$ = 56,0 dyn/cm (1 %ig in $H_2O$)
OH-Zahl: 136
Säurezahl: 50

Zur Bestimmung der Verseifungszahl wird eine 10 g-Probe des Produktes in 200 ml Ethanol gelöst und mit 1 g Raney-Nickel-Katalysator bei 100°C und 100 bar Wasserstoffdruck über Nacht im Autoklaven hydriert. Man erhält in quantitativer Ausbeute einen farblosen Feststoff.

Schmelzbereich: 65-68°C
OH-Zahl: 157
Säurezahl: 44
Verseifungszahl: 124

Beispiel 2

Gemäß Beispiel 1 werden 25,1 g wasserfreie Glucose und 125,5 g Stearinsäure (Molverhältnis 1:3,17) in 350 g vorgelegtem flüssigem Fluorwasserstoff gelöst und 1 Stunde bei 25°C gehalten. Nach der in Beispiel beschriebenen Aufarbeitung werden 121,5 g farbloses Festprodukt erhalten.

Schmelzbereich: 54-55°C
OH-Zahl: 122
Säurezahl: 160

Zur Bestimmung der Verseifungszahl wird wie in Beispiel 1 beschrieben, katalytisch hydriert.

Schmelzbereich: 53,0-55,5°C
OH-Zahl: 295
Säurezahl: 157
Verseifungszahl: 111

Beispiel 3

Gemäß Beispiel 1 werden 25,3 g wasserfreie Glucose, 25,3 g Myristinsäure, 25,3 g Palmitinsäure und 25,3 g Stearinsäure (Molverhältnis 1,27:1:0,89:0,80) in 236 g vorgelegtem flüssigen Fluorwasserstoff gelöst und 1 Stunde bei 25°C gerührt. Nach der in Beispiel 1 beschriebenen Aufarbeitung erhält man 78,6 g eines leicht gelblichen und klebrigen Festproduktes.

Schmelzbereich: 45 - 47°C

Zur Bestimmung der Verseifungszahl wird wie in Beispiel 1 beschrieben katalytisch hydriert.

Verseifungszahl: 103

Beispiel 4

In der in Beispiel 1 beschriebenen Apparatur wurden 95,6 g wasserfreie Glucose und 53,1 g Laurinsäure (Molverhältnis 2:1) in 347 g vorgelegtem flüssigem Fluorwasserstoff gelöst und 1 Stunde bei 25°C gehalten. Abweichend von der in Beispiel 1 beschriebenen Aufarbeitung wird nach weitgehender destillativer Entfernung des Fluorwasserstoffs der Rückstand mit ca. 70°C warmem Wasser aufgenommen, mit Calciumcarbonat neutralisiert (schäumt sehr stark) und die gesamte Mischung gefriergetrocknet. Das Festprodukt wird mit 2 1 Dimethylformamid aufgerührt, von den unlöslichen Calciumsalzen filtriert, das Filtrat am Dünnschichtverdampfer konzentriert, mit 1 1 Aceton gefällt und das Fällungsprodukt anschließend nochmals gründlich mit Aceton gewaschen. Man erhält 97,6 g farblosen Feststoff.

Schmelzbereich: 137-139°C

OH-Zahl: 535

Säurezahl: 10

Zur Bestimmung der Verseifungszahl wird wie in Beispiel 1 beschrieben katalytisch hydriert.

Schmelzbereich: 138-142°C

OH-Zahl: 613

Säurezahl: 7

Verseifungszahl: 156

Beispiel 5

In der in Beispiel 1 beschriebenen Apparatur werden 65,1 g wasserfreie Glucose und 32,6 g Stearinsäure (Molverhältnis 3,16:1) in 228 g vorgelegtem flüssigem Fluorwasserstoff gelöst und 1 Stunde bei 25°C gerührt. Abweichend von der in Beispiel 1 beschriebenen Aufarbeitung wird nach weitgehender destillativer Entfernung des Fluorwasserstoffs mit ca. 80°C heißem Dimethylformamid versetzt und mit Calciumcarbonat neutralisiert. Nach Filtration wird die DMF-Lösung am Dünnschichtverdampfer eingeengt, die konzentrierte Lösung mit 1 l Aceton versetzt und das Fällungsprodukt nach Absaugen nochmals gründlich mit Aceton gewaschen und getrocknet. Man erhält 71,5 g farbloses Pulver.

Schmelzbereich: 98-100,5°C

OH-Zahl: 450

Säurezahl: 16

Oberflächenspannung: $\delta$ = 40,6 dyn/cm (1 %ig in $H_2O$)

Zur Bestimmung der Verseifungszahl wird wie in Beispiel 1 beschrieben katalytisch hydriert. Man erhält einen farblosen Sirup.

OH-Zahl: 220

Säurezahl: 31

Verseifungszahl: 214

Oberflächenspannung: $\delta$ = 37,8 dyn/cm (1%ig in $H_2O$)

Beispiel 6

Gemäß Beispiel 1 werden 20,1 g wasserfreie Glucose und 60,3 g Palmitinsäure (Molverhältnis 1:2,1) in 188 g vorgelegtem flüssigem Fluorwasserstoff gelöst und 1 Stunde bei 25°C gehalten. Nach der in Beispiel 1 beschriebenen Aufarbeitung werden 63,2 g farbloses pulvriges Festprodukt erhalten.

Schmelzbereich: 52 - 54°C

OH-Zahl: 107

Säurezahl: 81

Zur Bestimmung der Verseifungszahl wird wie in Beispiel 1 beschrieben katalytisch hydriert.

OH-Zahl: 105

Verseifungszahl: 115

Beispiel 7

Gemäß Beispiel 1 werden 59,5 g wasserfreie Glucose und 89,2 g Laurinsäure (Molverhältnis 1:1,35) in 347 g vorgelegtem flüssigem Fluorwasserstoff gelöst und 1 Stunde bei 25°C gehalten. Nach der in Beispiel 5 beschriebenen Aufarbeitung erhält man 120,8 g eines leicht gelblichen Sirups.

OH-Zahl: 191

Säurezahl: 89

Zur Bestimmung der Verseifungszahl wird wie in Beispiel 1 beschrieben katalytisch hydriert. Man erhält einen leicht gelblichen Sirup.

OH-Zahl: 223

Säurezahl: 85

Verseifungszahl: 263

Beispiel 8

Gemäß Beispiel 1 werden 95,5 g Saccharose und 79,4 g Stearinsäure (Molverhältnis 1:1) in 408 g vorgelegtem flüssigem Fluorwasserstoff gelöst und 1 Stunde bei 25°C gerührt. Nach der in Beispiel 1 beschriebenen Aufarbeitung erhält man 72,0 g eines farblosen, körnigen Feststoffes.

Schmelzbereich: 63,5-65,0°C
OH-Zahl: 122
Säurezahl: 79

Beispiel 9

Abweichend von Beispiel 1 werden zunächst 137,1 g Fructose in 320 g vorgelegtem flüssigem Fluorwasserstoff gelöst und 1 Stunde bei 25°C gerührt, danach 216,6 g Stearinsäure (Molverhältnis 1:1) zugesetzt und noch eine weitere Stunde bei 25°C gehalten. Nach der im Beispiel 1 beschriebenen Aufarbeitung erhält man 224,8 g eines leicht gelblichen körnigen Feststoffs.

Schmelzbereich: 60-62°C
OH-Zahl: 83
Säurezahl: 74

Beispiel 10

Gemäß Beispiel 1 werden 155 g wasserfreie Glucose und 345 g Laurinsäure (Molverhältnis 1:2) in 500 ml vorgelegtem flüssigem Fluorwasserstoff gelöst und 1 Stunde bei 25°C gehalten. Nach der im Beispiel 1 beschriebenen Aufarbeitung erhält man 358,7 g eines leicht gelblichen, hochviskosen Sirups.

Säurezahl: 88

Zur Entfernung der freien Fettsäure wird das gesamte Produkt in 300 ml einer Mischung aus 96:4:0,5 Volumenteilen an Chloroform/Aceton/Eisessig gelöst und über Kieselgel (Merck, Kieselgel 60,Bst. Nr. 7734), angesetzt mit Chloroform/Aceton 96:4 (Volumenteile) als Laufmittel, chromatographiert. Nach 10-14 1 essigsäurefreiem Laufmittel erhält man 182,8 g Laurinsäure zurück; nach Wechsel des Laufmittels zu Aceton/Methanol (1:1 Volumenteile) werden nach ca. 5-8 1 151,1 g Kohlenhydratester eluiert (nach Einengen im Vakuum als leicht gelblicher Sirup).

OH-Zahl: 223
Säurezahl: 35

Beispiel 11

Gemäß Beispiel 1 werden 115,4 g wasserfreie Glucose und 385 g Laurinsäure (Molverhältnis 1:3) in 500 ml vorgelegtem flüssigen Fluorwasserstoff gelöst und 1 Stunde bei 25°C gehalten. Nach der in Beispiel 1 beschriebenen Aufarbeitung erhält man 397,6 g eines leicht gelblichen, hochviskosen Sirups.

Zur Entfernung der freien Fettsäure wird das gesamte Produkt in 300 ml einer Mischung aus 96:4:0,5 Volumenteilen an Chloroform/Aceton/Eisessig gelöst und über Kieselgel (Merck, Kieselgel 60, Best. Nr. 7734), angesetzt mit Chloroform/Aceton 96:4 (Volumenteile) als Laufmittel, chromatographiert. Nachdem die überschüssige Laurinsäure auf diese Weise vom Produkt abgetrennt war, wird das Laufmittel zu Aceton/Methanol (1:1 Volumenteile) gewechselt und man erhält nach ca. 5 - 8 1 135,8 g eluierten Kohlenhydratester (nach Einengen im Vakuum als leicht gelblicher Sirup).

OH-Zahl: 167
Säurezahl: 59

Beispiel 12 (Vergleichsbeispiel zu US-PS 3,551,464)

Gemäß Beispiel 1 werden 38,2 g Glycerin und 117,9 g Stearinsäure (Molverhältnis 1:1) in 364 g vorgelegtem flüssigem Fluorwasserstoff gelöst und 1 Stunde bei 25°C gerührt. Nach der in Beispiel 1 beschriebenen Aufarbeitung erhält man 98,8 g eines farblosen Sirups.

OH-Zahl: 257
Säurezahl: 2
Verseifungszahl: 83

Oberflächenspannung:     $\delta$ = 45,7 dyn/cm (1% in $H_2O$)
Zusammensetzung laut GC-Analyse: Monoglycerid/Diglycerid/Triglycerid wie 45-65:23-31:0-30 Gew.-%.

Beispiel 13

Gemäß Beispiel 1 werden 19,0 g wasserfreie Glucose und 45,3 g Laurinsäuremethylester (Molverhältnis 1:2) in 150 ml vorgelegtem flüssigem Fluorwasserstoff gelöst und 1 Stunde bei 25°C gerührt. Abweichend von der in Beispiel 1 beschriebenen Aufarbeitung wird nach weitgehender destillativer Entfernung des Fluorwasserstoffs mit 250 ml heißem Wasser aufgenommen, mit Eis abgekühlt und mit 3 mal 300 ml Methylenchlorid extrahiert, die gesammelten organischen Phasen mit Natriumsulfat getrocknet, filtriert und das Filtrat im Vakuum eingeengt. Man erhält 52,2 g eines leicht gelblichen Sirups, der durch Säulenchromatographie (Kieselgel, Laufmittel Methylenchlorid/Aceton 96:4 (Volumenanteile)) von überschüssigem Laurinsäuremethylester gereinigt wird (31,1 g). Das Kohlenhydratester-Produkt wird mit Aceton/Methanol (1:1, Volumenanteile) als Laufmittel eluiert. Man erhält 15,5 g eines leicht gelblichen, hochviskosen Sirups.

OH-Zahl:      222
Säurezahl:     35

Beispiel 14 (Anwendungsbeispiel)

Die Herstellung von Schokolade erfolgt gemäß folgendem Vorgehen:
1) Herstellung einer Mischung aus

| | |
|---|---|
| Puderzucker | 47,6 g |
| Kakao | 38,0 g |
| Kakaobutterersatz*/erfindungsgemäßer Fettersatzstoff aus Beispiel 10 bzw. 11 im Massenverhältnis 60:40 | 14,0 g |
| Lecithin | 0,4 g |

* Als Kakaobutterersatz wurde Cobein ex-Loders und Nucalin verwendet.

2) Schmelzen der Mischung
3) Abkühlen auf 27°C
4) leichtes Erwärmen auf 31°C und
5) sogleich in die Formen gießen. abkühlen lassen und lagern.
Alternativ kann auch von einer dicken Paste ausgegangen werden, die sich wie folgt zusammensetzt:

| | |
|---|---|
| 1) Puderzucker | 51,4 g |
| Kakao | 41,0 g |
| Kakaobutterersatz* / erfindungsgemäßer Fettersatzstoff aus Beispiel 10 bzw. 11 im Massenverhältnis 60:40 | 7,6 g |
| 2)-5) wie oben | |

* Als Kakaobutterersatz wurde Cobein ex-Loders und Nucalin verwendet.

In Tabelle 1 und 2 wird die Kalorienreduzierung ermittelt, die sich daraus errechnen läßt, daß man normalerweise verwendetes Fett teilweise oder ganz durch die erfindungsgemäßen Produkte ersetzt.

## Tabelle 1   Kalorische Daten der reinen Substanzen

| Zusammensetzung des Produkts [Einsatz-Molverhältnis] | Kalorischer Gehalt [kcal/g] | Reduzierung des kalorischen Gehalts [%] |
|---|---|---|
| Glucose:Laurinsäure (1:2) (Beispiel 10) | 7,37 | 18 |
| Glucose:Laurinsäure (1:3) (Beispiel 11) | 7,79 | 13 |
| reines Fett (zum Vergleich) | 9 | 0 |

## Tabelle 2   Kalorienreduzierung in Schokolade

| | Fett:Fettersatz-stoff (Massenverhältnis) | Kalorien-reduzierung [%] |
|---|---|---|
| Schokolade gemäß Beisp. 14 mit erfindungsgemäßem Produkt aus Beispiel 10 | 60:40 | 4,5 |
| Schokolade gemäß Beisp. 14 mit erfindungsgemäßem Produkt aus Beispiel 11 | 60:40 | 3,3 |
| theoretischer Wert bei vollständigem Fettersatz mit Produkt aus | | |
| a) Beispiel 10 | 0:100 | 10,0 |
| b) Beispiel 11 | 0:100 | 7,5 |
| ohne Verwendung von erfindungsgemäßen Fettersatzstoffen | 100:0 | 0,0 |

Dabei wird zunächst von der Annahme ausgegangen, daß die erfindungsgemäßen Substanzen vollständig metabolisiert werden. Bei gegebener Nichtmetabolisierbarkeit kann eine maximale Kalorienersparnis von 55 % erreicht werden.

Beispiel 15 (Anwendungsbeispiel)

Eine Testemulsion wurde wie folgt hergestellt: die unten angegebenen Mengen an [R]Hostaphat KW 340 N (Hersteller Hoechst AG)*und Paraffinöl-Mischung werden zusammen mit dem erfindungsgemäßen Produktgemisch auf 80°C erwärmt. Anschließend wurde die ebenfalls +80°C warme Wassermenge in die Öl- und Emulgator- bzw. Coemulgatormischung eingerührt.

| 5,0 Massenteile | Hostaphat KW 340 N |
| 20,0 Massenteile | Paraffinöl, hochviskos |
| 1,5 Massenteile | des erfindungsgemäßen Produktgemisches aus Beispiel 3 |
| ad 100,0 Massenteile | Wasser |

Die sich bildende Emulsion wird dann unter Rühren auf Raumtemperatur gebracht und nach 24stündiger Lagerzeit bei +20°C bezüglich ihrer Eigenschaften untersucht.

Mit Hilfe eines Rotationsviskosimeters (Fa. Haake RV 3, Karlsruhe) wurden die folgenden Viskositätswerte ermittelt:

Emulsion ohne erfindungsgemäßes Produktgemisch: niedrig viskos

Emulsion + 2 % erfindungsgemäßes Produktgemisch: 2990 mPas

Emulsion + 3 % erfindungsgemäßes Produktgemisch: 3025 mPas

Emulsion + 5 % erfindungsgemäßes Produktgemisch: 4825 mPas

Beispiel 16 (Anwendungsbeispiel)

| 0,5 Massenteile | erfindungsgemäßes Produktgemisch aus Beispiel 1 bzw. 3, |
| 9,5 Massenteile | Glycerinmonostearat |
| 15,0 Massenteile | Paraffinöl hochviskos |
| 10,0 Massenteile | Isopropylpalmitat |
| 0,18 Massenteile | Para-Hydroxybenzoesäuremethylester |
| 0,02 Massenteile | Para-Hydroxybenzoesäurepropylester |
| 0,2 Massenteile | Parfümöl |
| ad 100,0 Massenteile | destilliertes Wasser |

Die Herstellung der obigen Emulsion erfolgt durch Erwärmen der erfindungsgemäßen Produktgemische zusammen mit Glycerinmonostearat, Paraffinöl und Isopropylpalmitat auf +80°C. Anschließend wird Wasser mit der gleichen Temperatur in die heiße Mischung von Emulgator und Öl eingerührt und die sich bildende Emulsion unter kontinuierlichem Rühren auf Raumtemperatur gebracht.

Die Prüfung der physikalischen Lagerstabilität erfolgte durch mehrwöchige Lagerung der Emulsion bei +40 und in einem zweiten Versuch sogar bei +45°C.

Die nach obigem Verfahren formulierte O/W-Emulsion zeigte eine Lagerstabilität bei den genannten Testtemperaturen von über 2 Monaten, d.h. nach Ablauf dieser Zeit wurden visuell und mikroskopisch keine Instabilitätserscheinungen beobachtet.

## Patentansprüche

1. Verfahren zur Herstellung von Gemischen aus mono- und oligomeren Kohlenhydratestern durch Umsetzung von Kohlenhydraten mit mindestens einer gesättigten Fettsäurekomponente aus der Gruppe Fettsäuren, deren Anhydride, Halogenide und Ester, dadurch gekennzeichnet, daß man die Umsetzung in flüssigem Fluorwasserstoff als Reaktionsmedium urd Lösungsmittel durchführt und anschließend den Fluorwasserstoff abdestilliert.

2. Verfahren nach Anspruch 1, dadurch gekennzeichnet, daß die Umsetzurg bei Temperaturen von mindestens -40°C bis höchstens +80°C, vorzugsweise von -20°C bis +60°C und insbesondere von 0 bis +40°C durchgeführt wird, wobei die Reaktionszeit im allgemeinen 10 Minuten bis 6 Stunden, vorzugsweise 30 Minuten bis 3 Stunden und insbesondere 1 bis 2 Stunden beträgt urd die Zeit für das Abdestillieren des Fluorwasserstoffs nicht mit eingerechnet ist.

3. Verfahren nach Anspruch 1 oder 2, dadurch gekennzeichnet, daß die Gesamtmenge der Kohlenhydrate und der Fettsäurekomponente in der Fluorwasserstofflösung 10 bis 60, vorzugsweise 25 bis 35 Gew.-%, bezogen auf die Fluorwasserstofflösung, beträgt.

4. Verfahren nach einem oder mehreren der Ansprüche 1 bis 3, dadurch gekenrzeichnet, daß das molare

* Hostaphat KW 340 N enthält einen Polyglykolether-o-Phosphorsäureester.

Verhältnis der Kohlenhydrate zu der Fettsäurekomponente mindestens 1:5 und höchstens 10:1 ist.

5. Verfahren nach einem oder mehreren der Ansprüche 1 bis 4, dadurch gekennzeichnet, daß die Fettsäurekomponente 6-22, vorzugsweise 12-18 C-Atome hat und linear oder verzweigt, vorzugsweise jedoch geradzahlig ist und daß die Alkoholkomponente des Fettsäureesters ein einwertiger Alkohol mit bis zu 3 C-Atomen oder Glycerin ist.

6. Verfahren nach einem oder mehreren der Ansprüche 1 bis 5, dadurch gekennzeichnet, daß die Kohlenhydrate Aldohexosen oder davon abgeleitete Oligo- oder Polysaccharide, vorzugsweise Galactose, Mannose, Glucose oder davon abgeleitete Oligo- oder Polysaccharide sowie deren feste, trockene Pulvermischungen, oder Kohlenhydrate, die diese Aldohexosen als Bausteine enthalten, vorzugsweise Saccharose, oder Ketosen, vorzugsweise Fruktose sind.

7. Verfahren nach einem oder mehreren der Ansprüche 1 bis 6, dadurch gekennzeichnet, daß das Verfahrensprodukt nach dem Abdestillieren des Fluorwasserstoffs aufbereitet wird, indem es in einem geeigneten Lösungsmittel gelöst wird, mit einem geeigneten Ionenaustauscher oder Calciumlydroxid oder Calciumcarbonat versetzt wird, von dem Feststoff abgetrennt wird und dann entweder durch Einengung vom Lösungsmittel befreit wird oder durch Zusatz von geeigneten Fällungsmitteln ausgefällt und von der flüssigen Phase abgetrennt wird und daß zweckmäßig überschüssige Fettsäure chromatographisch abgetrennt wird.

8. Gemische aus mono- und oligomeren Kohlenhydratestern, erhältlich gemäß dem Verfahren nach einem oder mehreren der Ansprüche 1 bis 7.

9. Verwendung der Gemische aus mono- und oligomeren Kohlenhydratestern gemäß Anspruch 8 als Tenside.

10. Verwendung der Gemische aus mono- und oligomeren Kohlenhydratestern, erhältlich gemäß Verfahren nach Anspruch 7, als Fettersatzstoffe.

11. Verwendung der Gemische aus mono- und oligomeren Kohlenhydratestern, erhältlich gemäß Verfahren nach Anspruch 7, als Konsistenzgeber mit emulgierender und/oder koemulgierender Wirkung zur Herstellung von O/W-Emulsionen.

**Claims**

1. A process for the preparation of mixtures of mono- and oligomeric carbohydrate esters by reaction of carbohydrates with at least one saturated fatty acid component from the group comprising fatty acids, their anhydrides, halides and esters, which comprises carrying out the reaction in liquid hydrogen fluoride as reaction medium and solvent, and subsequently removing the hydrogen fluoride by distillation.

2. The process as claimed in claim 1, wherein the reaction is carried out at temperatures from at least -40°C to at most +80°C, preferably from -20°C to +60°C and, in particular, from 0 to +40°C, where the reaction time is generally 10 minutes to 6 hours, preferably 30 minutes to 3 hours and, in particular, 1 to 2 hours, and the time for removing the hydrogen fluoride by distillation is not included in the calculation.

3. The process as claimed in claim 1 or 2, wherein the total amount of carbohydrates and of fatty acid component in the hydrogen fluoride solution is 10 to 60, preferably 25 to 35% by weight based on the hydrogen fluoride solution.

4. The process as claimed in one or more of claims 1 to 3, wherein the molar ratio of the carbohydrates to the fatty acid component is at least 1:5 and at

5. The process as claimed in one or more of claims 1 to 4, wherein the fatty acid component has 6-22, preferably 12-18 carbon atoms, and is linear or branched, but is preferably even-numbered, and wherein the alcohol component of the fatty acid ester is a monohydric alcohol with up to 3 carbon atoms or glycerol.

6. The process as claimed in one or more of claims 1 to 5, wherein the carbohydrates are aldohexoses or oligo- or polysaccharides derived therefrom, preferably galactose, mannose, glucose or oligo- or polysaccharides derived therefrom, and their solid, dry powder mixtures, or carbohydrates which contain these aldohexoses as structural units, preferably sucrose, or ketoses, preferably fructose.

7. The process as claimed in one or more of claims 1 to 6, wherein the product of the process is, after removal of the hydrogen fluoride by distillation, treated in such a way that it is dissolved in a suitable solvent, a suitable ion exchanger or calcium hydroxide or calcium carbonate is added, the solid is separated off and then either solvent is removed by concentration, or precipitation is carried out by addition of suitable precipitants, and the liquid phase is separated off, and wherein excess fatty acid is expediently removed by chromatography.

8. A mixture of mono- and oligomeric carbohydrate esters obtainable by the process as claimed in one or more of claims 1 to 7.

9. The use of a mixture of mono- and oligomeric carbohydrate esters as claimed in claim 8 as surfactant.

10. The use of mixtures of mono- and oligomeric carbohydrate esters obtainable by the process as claimed in claim 7 as fat substitutes.

11. The use of mixtures of mono- and oligomeric carbohydrate esters obtainable by the process as claimed in claim 7 as bodying agents with emulsifying and/or coemulsifying action for the preparation of O/W emulsions.

**Revendications**

1. Procédé pour préparer des mélanges d'esters glucidiques monomères et oligomères par réaction de glucides avec au moins une composante d'acide gras saturé appartenant à l'ensemble constitué par les acides gras, leurs anhydrides, leurs halogénures et leurs esters, procédé caractérisé en ce qu'on effectue la réaction dans du fluorure d'hydrogène liquide comme milieu réactionnel et solvant et en ce qu'on chasse ensuite le fluorure d'hydrogène par distillation.

2. Procédé selon la revendication 1 caractérisé en ce que la réaction est effectuée à des températures comprises entre -40 °C et +80 °C, de préférence entre -20 °C et +60 °C et plus particulièrement entre 0 et +40 °C, la durée de la réaction étant généralement comprise entre 10 minutes et 6 heures, de préférence entre 30 minutes et 3 heures et, plus particulièrement, entre 1 et 2 heures, abstraction faite du temps nécessaire pour l'élimination du fluorure d'hydrogène par distillation.

3. Procédé selon la revendication 1 ou 2 caractérisé en ce que la quantité totale des glucides et de la composante d'acide gras dans la solution de fluorure d'hydrogène est comprise entre 10 et 60 % en poids, de préférence entre 25 et 35 % en poids, par rapport à la solution dans le fluorure d'hydrogène.

4. Procédé selon une ou plusieurs des revendications 1 à 3, caractérisé en ce que le rapport molaire des glucides à la composante d'acide gras est compris entre 1:5 et 10:1.

5. Procédé selon une ou plusieurs des revendications 1 à 4, caractérisé en ce que la composante d'acide gras renferme de 6 à 22 atomes de carbone, de préférence de 12 à 18, et est linéaire ou ramifiée, mais contient de préférence un nombre pair d'atomes de carbone, et en ce que la composante alcool de l'ester d'acide gras est un mono-alcool contenant au plus 3 atomes de carbone ou est le glycérol.

6. Procédé selon une ou plusieurs des revendications 1 à 5, caractérisé en ce que les glucides sont des aldohexoses ou des oligo- ou poly-saccharides qui en dérivent, de préférence le galactose, le mannose, le glucose ou des oligo- ou poly-saccharides qui en dérivent, ou des mélanges solides pulvérulents secs de ceux-ci, ou des glucides contenant de tels aldohexoses comme éléments constitutifs, de préférence le saccharose, ou des cétoses, de préférence le fructose.

13

**7.** Procédé selon une ou plusieurs des revendications 1 à 6, caractérisé en ce que le produit fourni par le procédé est soumis, après élimination du fluorure d'hydrogène par distillation, a un traitement complémentaire selon lequel on le dissout dans un solvant approprié, on ajoute un échangeur d'ions approprié ou de l'hydroxyde de calcium ou du carbonate de calcium, on sépare la matière solide, puis ou bien on élimine le solvant par concentration, ou bien on fait précipiter par addition d'agents de précipitation appropriés et on sépare la phase liquide, et on sépare avantageusement l'excès d'acide gras par chromatographie.

**8.** Mélanges d'esters glucidiques monomères et oligomères qui peuvent être obtenus par un procédé selon une ou plusieurs des revendications 1 à 7.

**9.** Application des mélanges d'esters glucidiques monomères et oligomères selon la revendication 8, comme surfactifs.

**10.** Application, comme substituts de corps gras, des mélanges d'esters glucidiques monomères et oligomères qui peuvent être obtenus par le procédé selon la revendication 7.

**11.** Application des mélanges d'esters glucidiques monomères et oligomères qui peuvent être obtenus par le procédé selon la revendication 7, comme donneurs de consistance ayant une action émulsionnante et/ou coémulsionnante pour la préparation d'émulsions LH (eau-dans-l'huile).